# EUROPEAN PATENT SPECIFICATION

(11) **EP 1 000 929 B1**
(45) Date of publication and mention of the grant of the patent: **29.12.2004**
(21) Application number: 98420185.5
(22) Date of filing: 13.10.1998
(51) Int. Cl.: C07C 253/14, C07C 255/50

(54) **Process for preparing o-nitrobenzonitriles**
Verfahren zur Herstellung von o-Nitrobenzonitrilen
Procédé de préparation de o-nitrobenzonitriles

(43) Date of publication of application: 17.05.2000
(73) Proprietor: Bayer CropScience S.A., 69009 Lyon (FR)
(72) Inventor: Viauvy, Agnès, 69700 Saint Andeol le Château (FR)
(74) Representative: Rippel, Hans Christoph, Dr.

(56) References cited:
- EP-A- 0 608 713
- EP-A- 0 613 719
- PATENT ABSTRACTS OF JAPAN vol. 015, no. 263 (C-0847), 4 July 1991 & JP 03 090057 A (ISHIHARA SANGYO KAISHA LTD), 16 April 1991
- DATABASE WPI Section Ch, Week 8133 Derwent Publications Ltd., London, GB; Class B05, AN 81-59682D XP002099154 & JP 56 079662 A (NIPPON KAYAKU KK) , 30 June 1981

## Description

This invention relates to novel processes for preparing intermediates (particularly 4-cyano-3-nitrobenzotrifluoride) useful in the preparation of pesticides.

Pesticidal 4-benzoylisoxazoles, particularly 5-cyclopropylisoxazole herbicides and intermediate compounds in their synthesis, are described in the literature, for example in European Patent Publication Nos. 0418175, 0527036, 0560482, 0609798 and 0682659.

Various methods for preparing these compounds are known, for example in European Patent Publication Nos. 0608713 and 0613719 and Japanese Patent Publication Nos. 3090057 and 56079662, which describe processes for preparing aromatic cyanides by reaction of an aromatic halide with an alkali cyanide. The present invention seeks to provide improved methods for the preparation of pesticides and the intermediate compounds useful in preparing them.

It is therefore an object of the present invention to provide a novel process for the preparation of ortho-nitrobenzonitrile compounds.

It is a further object of the present invention to provide a process for the preparation of ortho-nitrobenzonitrile compounds which proceeds with high selectivity.

The present invention allows these objects to be met in whole or in part.

### Summary of the Invention

The present invention accordingly provides a process for the preparation of an ortho-nitrobenzonitrile compound of formula (I): 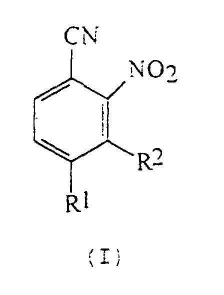 wherein:
R¹ represents C₁₋₄ haloalkyl (preferably trifluoromethyl), fluorine, chlorine or bromine; and R² represents hydrogen or C₁₋₄ alkoxy; which process comprises the reaction of the corresponding ortho-nitrofluorobenzene of formula (II): 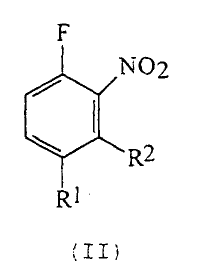
wherein R¹ and R² are as hereinbefore defined, with an alkali metal cyanide, in a non aqueous solvent optionally in the presence of a catalyst.

Certain compounds of formula (I) are known and a number of processes for their preparation and conversion into herbicidal 4-benzoylisoxazole derivatives have been described in the European Patent Applications cited above.

Compounds of formula (II) are known or may be prepared by known methods.

In formulae (I) and (II) and in the formulae depicted hereinafter, preferred values of the symbols are as follows:-

Preferably R¹ represents trifluoromethyl, fluorine or bromine; and R² represents hydrogen or methoxy.

In a particularly preferred embodiment of the invention R¹ represents trifluoromethyl and R² represents hydrogen.

### Detailed Description of the Invention

The above preparation of compounds of formula (I) from compounds of formula (II) is performed with an alkali metal cyanide (for example sodium cyanide or potassium cyanide). Sodium cyanide is preferred. The amount of cyanide used is generally from 1-2 molar equivalents, preferably from 1-1.1 molar equivalents.

A number of non-aqueous solvents are suitable, for example nitriles such as acetonitrile or benzonitrile; ethers such as tetrahydrofuran or diglyme (diethylene glycol dimethyl ether); amides such as N,N-dimethylformamide or N-methylpyrrolidone; ketones such as methyl isobutyl ketone; esters such as methyl benzoate or n-butyl acetate; dimethylsulphoxide or sulpholane. Preferably the solvent is chosen from benzonitrile, acetonitrile, tetrahydrofuran or N,N-dimethylformamide.

The reaction is generally conducted in a solvent with less than about 1% by volume water content, preferably less than about 0.5%, even more preferably less than about 0.1%, typically from about 0.005 to about 0.05%. It will however be understood that in certain cases slightly more or less water may be tolerated, depending on the nature of the solvents used and the temperature of the reaction, the compound of formula (I) to be prepared and other reaction conditions.

Preferably a catalyst is used, which may be selected from ammonium salts (such as tetraalkylammonium or trialkylbenzylammonium chlorides, bromides or hydrogen sulphate salts, in which the alkyl groups are straight- or branched-chain containing from one to six carbon atoms, such as tetramethylammonium bromide); or preferably guanidinium salts (such as hexabutylguanidinium chloride or hexamethylguanidinium chloride). The amount of catalyst when employed is generally from 0.01 to 0.3 molar equivalent, preferably from 0.05-0.25 molar equivalent.

Generally the reaction temperature is from 20°C to the boiling point of the solvent, preferably from 30°C to 180°C, and more preferably from 60°C to 100°C.

The compounds of formula (I) obtained by the process of the present invention may be used in the preparation of herbicidally active 4-benzoylisoxazole derivatives, for example according to the following reaction scheme:-

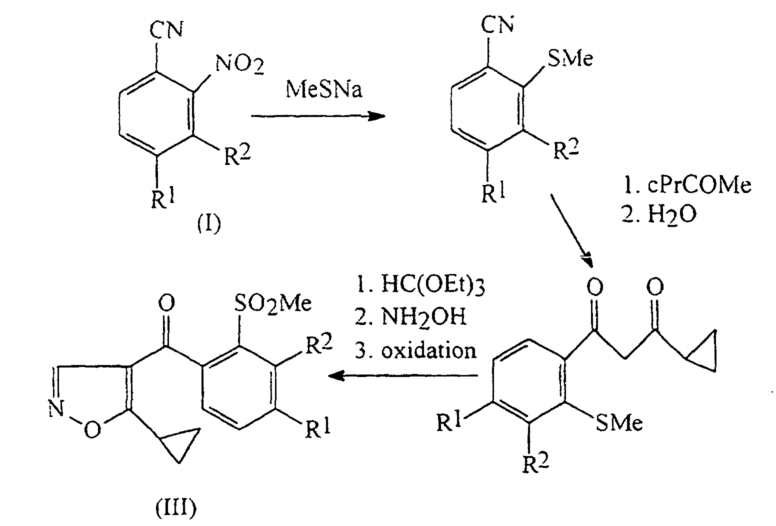

The 4-benzoylisoxazoles of formula (III) are described in for example European Patent Publication Nos. 0418175, 0527036 and 0560482.

The following non-limiting examples illustrate the invention.

### Example 1

### Preparation of 4-cyano-3-nitrobenzotrifluoride from 4-fluoro-3-nitrobenzotrifluoride using an alkali metal cyanide

A mixture of 4-fluoro-3-nitrobenzotrifluoride (1mmol) and sodium cyanide or potassium cyanide (1mmol), and acetonitrile or benzonitrile (1ml/mmol) were mixed at 20°C and heated for 6 hours at 80°C to give the title product. The results are shown in Table 1, from which it can be seen that the use of sodium cyanide gives good selectivity.

**Table 1**

| **Cyanide** | **Solvent** | **Conversion (%)** | **Yield (%)** | **Selectivity (%)** |
|---|---|---|---|---|
| NaCN | CH3CN | 79 | 45 | 57 |
| KCN | CH3CN | 76 | 38 | 49 |
| NaCN | PhCN | 45 | 36 | 80 |
| KCN | PhCN | 35 | 26 | 74 |

### Example 2

### Preparation of 4-cyano-3-nitrobenzotrifluoride from 4-fluoro-3-nitrobenzotrifluoride using sodium cyanide: effect of solvent, temperature and catalyst:

A mixture of 4-fluoro-3-nitrobenzotrifluoride (1mmol) and sodium cyanide (1mmol) and N,N-dimethylformamide, acetonitrile, tetrahydrofuran or benzonitrile (1ml/mmol) were mixed at 20°C and heated for 6 hours to give the title product. The results are shown in Table 2, from which it may be observed that benzonitrile (PhCN) gave the highest selectivity.

**Table 2**

| **Solvent** | **Temp (°C)** | **Conversion (%)** | **Yield (%)** | **Selectivity (%)** |
|---|---|---|---|---|
| DMF | 80 | 95 | 35 | 37 |
| DMF | 40 | 83 | 38 | 46 |
| DMF | 25-30 | 80 | 31 | 39 |
| CH3CN | 80 | 79 | 45 | 57 |
| THF | 80 | 69 | 39 | 56 |
| PhCN | 80 | 45 | 36 | 80 |

The above experimental procedure was repeated at 80°C but in the presence of a catalyst (0.2 equivalent) and acetonitrile, tetrahydrofuran or benzonitrile (1ml/mmol). The catalysts used were hexabutylguanidinium chloride or tetrabutylammonium bromide. The results (shown in Table 3) indicate that the use of hexabutylguanidinium chloride as catalyst gave very good selectivity especially in tetrahydrofuran or benzonitrile.

**Table 3**

| **Catalyst** | **Solvent** | **Time** | **Conversion (%)** | **Yield (%)** | **Selectivity (%)** |
|---|---|---|---|---|---|
| (a) | CH3CN | 13h | 47 | 33 | 70 |
| (a) | THF | 6h | 45 | 40 | 90 |
| (a) | PhCN | 6h | 42 | 40 | 95 |
| (b) | CH3CN | 13h | 88 | 47 | 54 |

| | | | | | |
|---|---|---|---|---|---|
| (a) = hexabutylguanidinium chloride | | | | | |
| (b) = tetrabutylammonium bromide. | | | | | |

## Claims

1. A process for the preparation of a compound of formula (I): 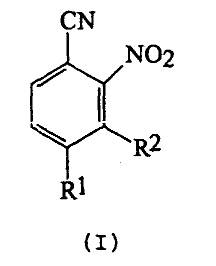 wherein:
R¹ represents C₁₋₄ haloalkyl, fluorine, chlorine or bromine; and R² represents hydrogen or C₁₋₄ alkoxy; which process comprises the reaction of the corresponding ortho-nitrofluorobenzene of formula (II): 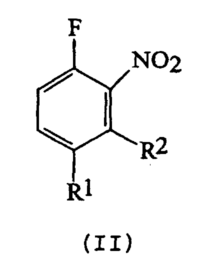
wherein R¹ and R² are as hereinbefore defined, with an alkali metal cyanide, in a non aqueous solvent which may have a water content of less than 1% by volume in the presence of a catalyst wherein the catalyst is selected from ammonium salts and guanidinium salts.

2. A process according to claim 1 in which the alkali metal cyanide is sodium cyanide.

3. A process according to claim 1 or 2 in which 1-2 molar equivalents of cyanide is used.

4. A process according to any one of the preceding claims in which the solvent is benzonitrile, acetonitrile, tetrahydrofuran or N,N-dimethylformamide.

5. A process according to any one of the preceding claims in which R¹ represents trifluoromethyl, fluorine or bromine; and R² represents hydrogen or methoxy.

6. A process according to any one of the preceding claims in which R¹ represents trifluoromethyl; and R² represents hydrogen.

## Patentansprüche

1. Verfahren zur Herstellung einer Verbindung mit der Formel (I) : 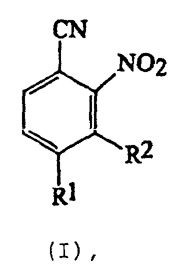 worin R¹ für C₁₋₄-Halogenalkyl, Fluor, Chlor oder Brom steht und R² Wasserstoff oder C₁₋₄-Alkoxy darstellt, welches Verfahren die Umsetzung des entsprechenden ortho-Nitrofluorbenzols mit der Formel (II): 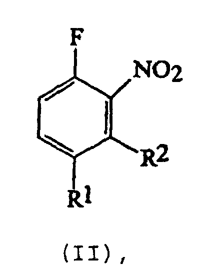 worin R¹ und R² wie vorstehend definiert sind, mit einem Alkalimetallcyanid in einem nichtwäßrigen Lösungsmittel, das einen Wassergehalt von unter 1 Vol.-% aufweisen kann, in Gegenwart eines Katalysators umfaßt, worin der Katalysator unter Ammoniumsalzen und Guanidiniumsalzen ausgewählt ist.

2. Verfahren nach Anspruch 1, worin das Alkalimetallcyanid Natriumcyanid ist.

3. Verfahren nach Anspruch 1 oder 2, worin 1 bis 2 Moläquivalente Cyanid verwendet werden.

4. Verfahren nach einem der vorstehenden Ansprüche, worin das Lösungsmittel Benzonitril, Acetonitril, Tetrahydrofuran oder N,N-Dimethylformamid ist.

5. Verfahren nach einem der vorstehenden Ansprüche, worin R¹ für Trifluormethyl, Fluor oder Brom steht und R² Wasserstoff oder Methoxy darstellt.

6. Verfahren nach einem der vorstehenden Ansprüche, worin R¹ Trifluormethyl bedeutet und R² Wasserstoff darstellt.

## Revendications

1. Procédé de préparation d'un composé de formule (I) : 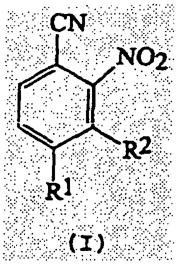 dans laquelle
R¹ représente un groupe haloalkyle en C₁-C₄, fluorine, chlorine ou bromine ; et
R² représente un atome d'hydrogène ou un groupe alkoxy en C₁-C₄ ; lequel procédé comprend la réaction de l'orthonitrofluorobenzène de formule (II) : 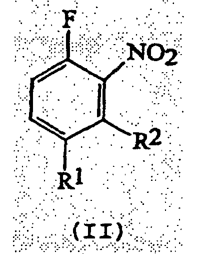
dans laquelle R¹ et R² sont tels que définis ci-dessus, avec un cyanure de métal alcalin, dans un solvant non-aqueux qui peut présenter un contenu en eau de moins de 1% en volume, en présence d'un catalyseur, le catalyseur étant sélectionné parmi les sels d'ammonium et les sels de guanidinium.

2. Procédé selon la revendication 1, dans lequel le cyanure de métal alcalin est le cyanure de sodium.

3. Procédé selon la revendication 1 ou 2, dans lequel 1 à 2 équivalents molaires de cyanure est utilisé.

4. Procédé selon l'une quelconque des revendications précédentes, dans lequel le solvant est le benzonitrile, l'acétonitrile, le tétrahydrofurane ou la N,N-diméthylformamide.

5. Procédé selon l'une quelconque des revendications précédentes, dans lequel R¹ représente un groupe trifluorométhyle, fluorine ou bromine ; et R² représente un atome d'hydrogène ou un groupe méthoxy.

6. Procédé selon l'une quelconque des revendications précédentes, dans lequel R¹ représente un groupe trifluorométhyle ; et R² représente un atome d'hydrogène.
